# EUROPEAN PATENT APPLICATION

(11) **EP 3 598 138 A1**
(43) Date of publication of application: **22.01.2020**
(21) Application number: 19382413.3
(22) Date of filing: 22.05.2019
(51) Int. Cl.: G01N 33/68, G01N 33/50

(54) **METHOD FOR THE DIAGNOSTIC AND/OR PROGNOSTIC ASSESSMENT OF ACUTE-ON-CHRONIC LIVER FAILURE SYNDROME IN PATIENTS WITH LIVER DISORDERS**

(71) Applicant: European Foundation for the Study of Chronic Liver Failure (EF-CLIF), 08021 Barcelona (ES)
(72) Inventor: JUNOT, Christophe, 92170 VANVES (FR); FENAILLE, Francois, 91440 BURES SUR YVETTE (FR); MOREAU, Richard, 95880 ENGHIEN LES BAINS (FR); ARROYO PEREZ, Vicente, 08021 BARCELONA (ES); CLARIA ENRICH, Joan, 08635 SANT ESTEVE SESROVIRES (ES)
(74) Representative: Durán-Corretjer, S.L.P.

(57) **Abstract**

The present invention discloses an *ex vivo* method for the diagnostic and/or prognostic assessment of the acute-on-chronic liver failure (ACLF) syndrome in a patient with a liver disorder characterized in that it comprises the steps of: (a) measuring a panel of metabolites related with acylcarnitines-sialic acid-acetylated amino acids and/or sugar alcohols and derivatives-tryptophan metabolism-catecholamines derivatives in a biological sample of said patient; and (b) comparing the level of said metabolites in the sample with the level of said metabolites in healthy patients; and wherein an increase of at least 1.2 times of the level of said metabolites is indicative of ACLF syndrome.

## Description

The present invention relates to the diagnostic and prognostic field. In particular, it refers to diagnostic and/or prognostic of the acute-on-chronic liver failure (ACLF) syndrome in a patient with a liver disorder by the assessment of specific serum metabolites.

Liver disorders occur when large parts of the liver become damaged beyond repair and the liver is no longer able to function. Liver failure is a life-threatening condition that demands urgent medical care. Most often, liver failure occurs gradually and over many years. The most common causes of chronic liver failure include hepatitis B, hepatitis C, long-term alcohol consumption and cirrhosis.

Cirrhosis is a pathological diagnosis characterised by diffuse fibrosis, severe disruption of the intrahepatic arterial and venous flow, portal hypertension and, ultimately, liver failure. Traditionally, cirrhosis has been dichotomised in to two stages: compensated and decompensated. Compensated cirrhosis (CC), defined as the period between the onset of cirrhosis and the appearance of the first major complication of the disease. During this period, which is relatively long in most patients (>10 years), symptoms are absent or minor but liver lesions steadily progress. The term decompensated cirrhosis defines the period following the development of ascites, variceal bleeding and hepatic encephalopathy. This period is associated to short-term survival (3-5 years).

Modern medical research and the introduction of a new and effective therapies for some etiological forms of cirrhosis, particularly antiviral drugs, have determined major changes on our concepts on the disease. First, cirrhosis is no longer considered to be an irreversible progressive disease. Indeed, decompensated cirrhosis may return to compensated cirrhosis or even to pre-cirrhotic phases if the cause of the disease is removed. Second, the initial list of organ or system dysfunctions in cirrhosis (liver, kidneys, brain) has been expanded to include the immune system, intestine, heart, lungs, adrenal glands, muscles, and thyroid gland. Third, new mechanisms involved in the pathogenesis of cirrhotic complications, such as dysbiosis of the microbiota and systemic inflammation, have been recognized. Last, it is increasingly evident that patients rarely die as a consequence of an end-stage irreversible destruction of the liver. Rather, in most patients the cause of death is an acute deterioration in their clinical condition promoted by a precipitating event, a syndrome term ACLF syndrome.

ACLF syndrome is a recently recognized syndrome characterized by acute decompensation (AD) of cirrhosis, and associated with different combinations of organ failures among the six major organ systems (liver, kidney, brain, coagulation, circulation, and respiration), giving rise to different clinical phenotypes (Moreau R, et al. Acute-on-chronic liver failure is a distinct syndrome developing in patients with acute decompensation of cirrhosis. Gastroenterology 2013;144:1426-37). ACLF syndrome can be triggered by a precipitating event (e.g. bacterial infection, active alcoholism) and is invariably associated with exacerbated systemic inflammation. The applicant has found that the patients with ACLF syndrome can be classified into three groups, essentially according to the number of impaired organs based on three assumptions and on a pragmatic assessment of the clinical features, course and prognosis (28-day and 90-day survival) of the patients included in the CANONIC study (Moreau R, et al. Acute-on-chronic liver failure is a distinct syndrome developing in patients with acute decompensation of cirrhosis. Gastroenterology 2013;144:1426-37). Based on these criteria, patients with decompensated cirrhosis can be stratified into four groups of severity:
1) No ACLF. This group comprises 3 subgroups: (1) patients with no organ failure, (2) patients with a single "non-kidney" organ failure (ie, single failure of the liver, coagulation, circulation, or respiration) who had a serum creatinine level <1.5 mg/dL and no hepatic encephalopathy, and (3) patients with single cerebral failure who had a serum creatinine level <1.5 mg/dL.
2) ACLF grade 1. This group includes 3 subgroups: (1) patients with single kidney failure, (2) patients with single failure of the liver, coagulation, circulation, or respiration who had a serum creatinine level ranging from 1.5 to 1.9 mg/dL and/or mild to moderate hepatic encephalopathy, and (3) patients with single cerebral failure who had a serum creatinine level ranging from 1.5 to 1.9 mg/dL.
3) ACLF grade 2. This group includes patients with 2 organ failures; and
4) ACLF grade 3. This group includes patients with 3 organ failures or more.

ACLF is a very dynamic syndrome. It may improve, follow a steady course or worsen during hospitalization. Although there is considerable variability between patients, some broad principles regarding the course of the condition can be put forward. The first is that improvement to no ACLF (resolution of ACLF) during hospitalization is frequent in patients with ACLF grade 1 (54%), relatively frequent in patients with ACLF grade 2 (35%) and infrequent (16%) in patients with ACLF grade 3 (Gustot T, et al. Clinical course of acute-on-chronic liver failure syndrome and effects on prognosis. Hepatology. 2015;62:243-252). The second is that few patients with ACLF grade 1 (21%) progress to ACLF grade 2 or grade 3. The third is that most patients with ACLF grade 2 (51%) follow either a steady course (26%) or progress to ACLF grade 3 (26%). The fourth is that most patients with ACLF grade 3 (68%) do not improve with the current medical treatment. Finally, the fifth is that 28-day and 90-day mortalities correlate with clinical course.

ACLF is very frequent, affecting 30%-40% of hospitalized patients, and is associated with high short-term mortality rate (30% at 28 days). However, the intrinsic mechanisms of ACLF at the tissue and cellular levels that contribute to the development and maintenance of organ failures are unknown. Understanding these mechanisms should not only result in progresses in the knowledge on pathophysiology of ACLF, but also could provide clues to the development of new biomarkers of organ dysfunction/failure and identification of targets for new therapies of organ failures which are urgently needed.

ACLF in cirrhosis frequently develops in the setting of an acute event that acts as a precipitating factor. The most frequent precipitating event of ACLF in Europe and North America are bacterial infections and acute alcoholic hepatitis. ACLF usually occurs in young cirrhotic, frequently alcoholics, in relation to a systemic inflammatory reaction due to bacterial infections, acute alcoholic liver injury or, in 40% of patients, to as yet unidentified precipitating events (Claria J, at al. Systemic inflammation in decompensated cirrhosis. Characterization and role in acute-on-chronic liver failure. Hepatology 2016; 64: 1249-1264). In Asia, acute viral hepatitis type A, B and E superimposed to cirrhosis are also common (Sarin SK, et al. Acute-on-chronic liver failure: terminology, mechanisms and management. Nat Rev Gastroenterol Hepatol 2016; 13: 131-149; Shi Yu, et al. Acute-on-chronic liver failure precipitated by hepatic injury is distinct from that precipitated by extrahepatic insults. Hepatology 2015; 62: 232-242).

ACLF can develop at any stage from compensated to decompensated cirrhosis (Moreau R, et al. Acute-on-chronic liver failure is a distinct syndrome developing in patients with acute decompensation of cirrhosis. Gastroenterology 2013;144:1426-37). In approximately 25% there is no prior history of acute decompensation (AD). In these patients, therefore AD associated to ACLF is the initial complication of cirrhosis. In other 25% of patients ACLF develops within a very short period (3 months) after the first episode of AD. Therefore, ACLF is frequently a complication of an early cirrhosis.

The clinical course and prognosis of ACLF depends partially on the presence and type of precipitating event. ACLF caused or complicated by infection shows a worse prognosis than that observed in ACLF patients without infection.

The estimated global cirrhosis deaths in 2010 was 1,029,042, (1.95% of the total global deaths). By comparison, in 2010 there were an estimated 714,492 colorectal cancer deaths, 752,100 liver cancer deaths (most of them developing in cirrhotic livers), 1,527,260 deaths from lung cancer and 5,870,446 deaths from cerebrovascular disease. Regional mortality rate depends on the prevalence of alcoholism, chronic hepatitis B and C viral infection and obesity (the most frequent causes of cirrhosis worldwide). In terms of aged-standardized mortality rates, the global mortality from cirrhosis in 2010 was 20.0 per 100.000 inhabitants (Mokdad AA, et al. Liver cirrhosis mortality in 187 countries between 1980 and 2010: a systematic analysis. BMC Medicine 2014; 12: 145).

There are clear data on the existence of at least two different subtypes of ACLF. Subtype 1 is predominant in Europe and in other regions (Republic of Korea) in which the main etiology of cirrhosis is alcohol (Li H, et al. Characteristics, diagnosis and prognosis of acute-on-chronic liver failure in cirrhosis associated to hepatitis B. Sci.Rep 2016;6,25487; Arroyo v, et al. Acute-on-chronic liver failure in cirrhosis. Nat Rev Dis Primers 2016;2:16041). Subtype 2 is the predominant subtype in China where chronic hepatitis B virus infection is by far, the main cause of cirrhosis (Kim TY, et al. Characteristics and discrepancies of acute-on-chronic liver failure: need for a Unified definition. PLos ONE2016;11(1):e0146745). The predominant organ/system failure in Subtype 1 is the kidney, and in subtype 2 the liver and coagulation. Severity of the syndrome, estimated by the mortality associated with each ACLF grade, is higher in subtype 2. In addition, the frequency of ACLF-2 and 3 at the onset of the syndrome is greater in subtype 2 than in subtype 1.

Systemic inflammation is associated with rapid and widespread catabolism including glycogenolysis (liver), proteolysis (mainly muscles) and lipolysis (adipose tissue) induced by cortisol, glucagon, adrenaline and inflammatory mediators, and secondary release of glucose, amino acids and fatty acids (FAs) to the circulation, to fuel the highly-demanding energetic process represented by the immune response (production of inflammatory mediators, immune cell proliferation, adhesion and migration, respiratory burst and production of acute-phase proteins) (O'Neill LA, Pearce EJ. Immunometabolism governs dendritic cell and macrophage function. J Exp Med. 2016 Jan 11 ;213(1):15-23; Van Wyngene L, Vandewalle J, Libert C. Reprogramming of basic metabolic pathways in microbial sepsis: therapeutic targets at last? EMBO Mol Med 2018;10. pii: e8712; Wang A, Luan HH, Medzhitov R. An evolutionary perspective on immunometabolism. Science 2019;363: eaar3932; Ganeshan K, Nikkanen J, Man K, et al. Energetic Trade-Offs and Hypometabolic States Promote Disease Tolerance. Cell 2019;177:399-413.e12). Therefore, in ACLF, systemic inflammation should lead to profound changes in most metabolic pathways.

Metabolomics, which identifies and quantifies small-molecule metabolites (the metabolome), is the omics which is the closest to clinical phenotypes (Guijas C, Montenegro-Burke JR, Warth B, et al. Metabolomics activity screening for identifying metabolites that modulate phenotype. Nat Biotechnol 2018;36:316-320). Interestingly, metabolites not only reflect the metabolic activity of tissues but, because many metabolites have powerful biological activity on critical pathophysiological processes, they also can influence the clinical phenotype. The best approach to characterize the metabolomic changes of such complex syndromes as ACLF, is to perform high throughput untargeted (agnostic) studies in large series of patients. This approach, which has been frequently applied to other diseases associated with systemic inflammation, including sepsis, has so far not been applied in the investigation of ACLF syndrome.

Some studies should be aimed at determining whether ACLF can be prevented and determining which patient is at risk for death, which patient will benefit from intensive care alone, which patient should have early liver transplant, and the role of regenerative therapies and bioartificial liver support. It is equally important, because ACLF is such an expensive disease to treat and so as not to waste resources, that it will be able to determine early in which patients treatment is likely to be futile.

As described above, ACLF is associated with high short-term mortality. ACLF grade at diagnosis is associated with short-term prognosis, with patients with ACLF grade 3 showing the worst prognosis compared with that of patients with ACLF grade 1 and 2. Therefore, it is very important to stratify patients according to prognosis, in order to monitor treatment responsiveness, determine emergency for transplantation, decide allocation in the intensive care unit and also to have a rational basis to decide futility.

Moreover, investigations of ACLF are generally performed after diagnosis. Therefore, identification of biomarkers or panels of biomarkers that could be used as predictors of said disease and survival is of considerable interest. In order to provide a solution to this issue, the inventors of the present invention has surprisingly found that measuring a panel of metabolites it is possible to know or predict ACLF in patients with a liver disorder and even more it is possible to identifying which organ will fail. In a first aspect, the present invention discloses an *ex vivo* method for diagnostic and/or prognostic of the acute-on-chronic liver failure (ACLF) syndrome in a patient with a liver disorder characterized in that it comprises the steps of: (a) measuring a panel of metabolites related with acylcarnitines-sialic acid-acetylated amino acids and/or sugar alcohols-tryptophan metabolism-acetylated amino acids in a biological sample of said patient; (b) comparing the level of said metabolites in the sample with the level of said metabolites in healthy patients, in which an increase of at least 1.2 times of the level of said metabolites is indicative of ACLF syndrome.

Preferably, said acylcarnitines-sialic acid-acetylated amino acids are 2-Heptanone, 5'-Deoxy-5'-(methylthio)adenosine, cystathionine, hexanoylcarnitine, N-Acetyl-L-tryptophan, phenol, N-Acetylneuraminic acid, N-Acetyl-L-phenylalanine, 2,2'-Thiodiacetic acid, kynurenic acid, octanoylcarnitine, trisaccharides, hydroxyphenylacetic acids, N6,N6,N6-Trimethyl-L-lysine, N-Acetyl-L-tyrosine, butyrylcarnitine, p-Hydroxyphenyllacticacid, decanoylcarnitine, N-Acetyl-L-aspartic acid.

Preferably, said sugar alcohols and derivatives-tryptophan metabolism-catecholamines derivatives are N-Acetyl-L-alanine, pentose alcohols, N-Acetyl-Asp-Glu, L-saccharopine, D-Galacturonic acid, quinolinic acid, beta-Pseudouridine, 4-Acetamidobutanoic acid, D-glucuronic acid, 4-Hydroxy-3-methoxyphenylglycol sulfate, D-Threitol, disaccharides, mevalonic acid, orotidine. These metabolites indicate changes in intracellular glucose utilization -, and quinolinic acid - a precursor for NAD+ biosynthesis.

In the method of the present invention said biological sample can be saliva, whole blood, plasma, serum, urine, among other biological fluids of a patient from which a genetic profile can be performed. Preferably, said biological sample is plasma or serum.

Moreover, with the method of the present invention it is possible to predict as well the development of organ failure. Preferably, said organ is liver, brain or kidney.

Preferably, an increased level of 5'-Deoxy-5'-(methylthio)adenosine, phenol, N6,N6,N6-Trimethyl-L-lysine, N-Acetyl-L-tyrosine, butyrylcarnitine, p-Hydroxyphenyllacticacid, pentose fosfates, D-Galacturonic acid or D-glucuronic acid is indicative of liver failure.

Preferably, an increased level of 2-Heptanone, trisaccharides, N-Acetyl-L-aspartic acid, N-Acetyl-Asp-Glu or L-saccharopine is indicative of brain failure.

Preferably, an increased level of cystathionine, hexanoylcarnitine, N-Acetyl-L-tryptophan, N-Acetylneuraminic acid, N-Acetyl-L-phenylalanine, 2,2'-Thiodiacetic acid, kynurenic acid, octanoylcarnitine, hydroxyphenylacetic acids, decanoylcarnitine, N-Acetyl-L-alanine, pentose alcohols, quinolinic acid, beta-Pseudouridine, 4-Acetamidobutanoic acid, 4-Hydroxy-3-methoxyphenylglycol sulfate, D-Threitol, disaccharides, mevalonic acid or orotidine is indicative of kidney failure.

Hereinafter, the present invention is described with reference to examples, which however are not intended to limit the present invention.

### EXAMPLES

EXAMPLE 1: Characterization of the patients with acutely decompensated cirrhosis whose biobanked serum samples have been used for metabolomics.

The study used biobanked serum samples from patients with acutely decompensated cirrhosis (334 with AD (without ACLF); 179 with ACLF) prospectively enrolled in the CANONIC study, along with 20 patients with compensated cirrhosis, and 29 healthy subjects. All these individuals or their legal representatives and the ethics committee of each hospital involved in the study gave informed consent for omics investigations in the biobanked material. Values for physiological variables and plasma cytokines and chemokines, neutrophil gelatinase-associated lipocalin (NGAL), soluble CD206 and soluble CD163 given in the manuscript were among those previously reported in other studies. 1,2,8-10 The clinical and standard laboratory characteristics of the patients included in the current investigation are illustrated in Table 1.

Samples of serum from patients with decompensated cirrhosis with and without ACLF were analyzed by liquid chromatography coupled to high resolution mass spectrometry (LC-HRMS). Two complementary methods involving both HILIC and C18 columns for metabolomics and C8 column for lipidomics were used in both positive and negative ionization modes (Boudah et al, J Chrom B 2014; Seyer et al, Metabolomics 2016).

**Table 1. Characteristics of the AD and ACLF patients.**

| **Characteristic** | **AD (n=334)** | **ACLF (n=179)** | **P value** | **ACLF-1 (n=95)** | **ACLF-2 (n=65)** | **ACLF-3 (n=19)** | ***P*-value** |
|---|---|---|---|---|---|---|---|
| **Demographical and clinical data** | | | | | | | |
| Age, y, mean ± SD | 57.1 ± 11.5 | 56.9 ± 11.3 | 0.85 | 58.7 ± 11.5 | 55.5 ± 10.9 | 52.6 ±- 9.9 | 0.05 |
| Male sex, n (%) | 221 (66.2) | 117 (65.4) | 0.85 | 65 (68.4) | 43 (66.1) | 9 (47.4) | 0.21 |
| Ascites, n (%) | 187 (56.2) | 130 (73.0) | <0.01 | 64 (67.4) | 51 (79.7) | 15 (78.9) | 0.19 |

| **Potential precipitating events of ACLF, n (%)** | | | | | | | |
|---|---|---|---|---|---|---|---|
| Bacterial Infection | 77 (23.1) | 62 (35.0) | <0.01 | 28 (29.8) | 22 (34.4) | 12 (63.2) | 0.02 |
| Active alcoholism | 37 (11.1) | 32 (17.9) | 0.07 | 11 (11.6) | 16 (24.6) | 5 (26.3) | 0.12 |
| Other precipitating event | 8 (2.4) | 13 (7.3) | <0.01 | 5 (5.3) | 7 (11.8) | 1 (5.7) | 0.39 |
| No precipitating event | 149 (44.6) | 74 (43.3) | 0.58 | 48 (50.5) | 22 (33.9) | 15 (78.9) | 0.03 |

| **Organ failures, n (%)** | | | | | | | |
|---|---|---|---|---|---|---|---|
| Liver | 36 (10.8) | 71 (39.7) | <0.01 | 21 (22.1) | 36 (55.4) | 14 (73.7) | <0.01 |
| Kidney | 0.00 (0) | 103 (57.5) | <0.01 | 61 (64.2) | 27 (41.5) | 15 (78.9) | <0.01 |
| Brain | 8 (2.4) | 35 (19.6) | <0.01 | 3 (3.2) | 21 (32.3) | 11 (57.9) | <0.01 |

| **Characteristic** | **AD (n=334)** | **ACLF (n=179)** | ***P* value** | **ACLF-1 (n=95)** | **ACLF-2 (n=65)** | **ACLF-3 (n=19)** | **P-value** |
|---|---|---|---|---|---|---|---|
| Coagulation | 12 (3.6) | 44 (24.6) | <0.01 | 6 (6.3) | 26 (40.0) | 12 (63.2) | <0.01 |
| Circulation | 4 (1.2) | 29 (16.2) | <0.01 | 1 (1.1) | 14 (21.5) | 14 (73.7) | <0.01 |
| Lungs | 3 (0.9) | 14 (7.8) | <0.01 | 3 (3.2) | 6 (9.2) | 5 (26.3) | <0.01 |

| **Organ dysfunctions, n (%)** | | | | | | | |
|---|---|---|---|---|---|---|---|
| Liver | 118 (35.3) | 102 (57.0) | <0.01 | 39 (41.0) | 48 (73.9) | 15 (78.9) | <0.01 |
| Kidney | 60 (18.0) | 118 (65.9) | <0.01 | 72 (75.8) | 32 (49.2) | 14 (73.7) | <0.01 |
| Brain | 88 (26.4) | 104 (58.1) | <0.01 | 52 (54.7) | 36 (55.4) | 16 (84.2) | 0.05 |

| **Mortality, n (%)** | | | | | | | |
|---|---|---|---|---|---|---|---|
| 28-day Mortality | 22 (6.6) | 48 (26.8) | <0.01 | 17 (17.9) | 18 (27.7) | 13 (68.4) | <0.01 |
| 90-day Mortality | 55 (16.5) | 72 (40.2) | <0.01 | 29 (30.5) | 30 (46.2) | 13 (68.4) | <0.01 |

| **Physiological variables** | | | | | | | |
|---|---|---|---|---|---|---|---|
| Serum bilirubin, mg/dL, mean ± SD | 5.6 ± 6.5 | 11.3 ± 11.4 | <0.01 | 6.8 ± 8.0 | 14.2 ± 10.7 | 24.4 ± 14.9 | <0.01 |
| International normalized ratio, mean ± SD | 1.6 ± 0.4 | 2.0 ± 0.9 | <0.01 | 1.6 ± 0.5 | 2.4 ± 0.9 | 2.9 ± 1.2 | <0.01 |

| **Median values of aminotransferases (IQR), U/L** | | | | | | | |
|---|---|---|---|---|---|---|---|
| Alanine aminotransferase | 36.0 (24-59.5) | 35 (22-57) | 0.71 | 30 (18-48) | 37.5 (28-54) | 58 (31-129) | <0.01 |
| Aspartate aminotransferase | 59.5 (38-102) | 67 (37-111) | 0.47 | 49.5 (34-93) | 76 (46-111) | 122.5 (89-149) | <0.01 |
| Serum creatinine, mg/dL, mean ± SD | 1.0 ± 0.4 | 2.3 ± 1.5 | <0.01 | 2.5 ± 1.5 | 1.9 ± 1.5 | 2.9 ± 1.9 | <0.01 |
| Blood white-cell count, x10⁹/L, mean ± SD | 7.4 ± 4.5 | 9.9 ± 7.1 | <0.01 | 8.2 ± 4.8 | 11.2 ± 7.0 | 14.6 ± 13.2 | <0.01 |
| Median levels of blood glucose (IQR), mg/dL | 107 (90-144) | 108 (87-142) | 0.71 | 107 (89-153) | 107 (86-133) | 116 (85-153) | 0.74 |

### EXAMPLE 2: Characterization of patient's metabolic profile associated with ACLF.

The aim of the study was to characterize the metabolic profile associated with ACLF, to assess if there was a blood metabolite fingerprint specific for ACLF of any grade and each of the three ACLF grades of severity.

Among the 153 annotated metabolites identified, 44 were increased in ACLF of any grade, relative to patients with acute decompensation. These 44 metabolites were distributed among two distinct panels which compose a specific blood metabolite fingerprint.

The two panels of metabolites are useful for the diagnosis of ACLF. The prototypic metabolites identified in this fingerprint are illustrated in the following tables.

Panel 1: Acylcarnitines-sialic acid-acetylated amino acids such as 2-Heptanone, 5'-Deoxy-5'-(methylthio)adenosine, cystathionine, hexanoylcarnitine, N-Acetyl-L-tryptophan, phenol, N-Acetylneuraminic acid, N-Acetyl-L-phenylalanine, 2,2'-Thiodiacetic acid, kynurenic acid, octanoylcarnitine, trisaccharides, hydroxyphenylacetic acids, N6,N6,N6-Trimethyl-L-lysine, N-Acetyl-L-tyrosine, butyrylcarnitine, p-Hydroxyphenyllacticacid, decanoylcarnitine, N-Acetyl-L-aspartic acid.

ACLF associates with increased proteolysis and lipolysis. As expected in the context of acute inflammation, several members of ACLF-associated subclusters were amino acids or amino acid metabolites indicating increased proteolysis (highlighted by increased N6,N6,N6-trimethyl-L-lysine levels; subcluster-A) indicating depression in mitochondrial β-oxidation and energy production - and N6,N6,N6-trimethyl-L-lysine - a lysine catabolite, precursor of carnitine.

**Table 2. Panel 1: Acylcarnitines-sialic acid-acetylated amino acids associated with ACLF.**

| **Metabolites** | **Healthy subjects (HS)** | **Compensated Cirrhosis (CC)** | **Acute Descompensation (AD)** | **Acute-on-chronic liver failure (ACLF)** |
|---|---|---|---|---|
| 2-Heptanone | 11,291.95 | 6,005.45 | 15,327.07 | 143,324.54 |
| 5'-Deoxy-5'-(methylthio)adenosine | 12,015.57 | 11,110.15 | 24,192.91 | 65,728.35 |
| Cystathionine | 6,500.29 | 16,008.09 | 40,565.34 | 100,677.70 |
| Hexanoylcarnitine | 138,607.31 | 120,414.57 | 219,245.01 | 454,397.92 |
| N-Acetyl-L-tryptophan | 30,119.16 | 36,242.58 | 36,475.55 | 580,509.30 |
| Phenol | 183,076.52 | 72,470.53 | 85,764.68 | 268,844.64 |
| N-Acetylneuraminic acid | 116,250.73 | 129,548.32 | 236,226.57 | 403,440.93 |
| N-Acetyl-L-phenylalanine | 62,597.58 | 91,076.14 | 129,801.86 | 204,762.91 |
| 2,2'-Thiodiacetic acid | 34,504.82 | 55,390.90 | 74,953.88 | 176,476.72 |
| Kynurenic acid | 148,269.72 | 136,022.66 | 140,300.20 | 277,842.52 |
| Octanoylcarnitine | 607,225.16 | 561,025.02 | 557,522.01 | 1,039,639.30 |
| Trisaccharides | 48,400.73 | 32,633.80 | 44,207.10 | 80,754.52 |
| Hydroxyphenylacetic acids | 135,386.99 | 330,658.26 | 545,979.52 | 1,603,551.28 |
| N6,N6,N6-Trimethyl-L-lysine | 221,161.51 | 222,285.30 | 281,148.73 | 444,299.51 |
| N-Acetyl-L-tyrosine | 49,264.06 | 87,082.65 | 133,573.52 | 252,120.01 |
| Butyrylcarnitine | 188,421.72 | 218,044.64 | 298,129.37 | 500,511.78 |
| p-Hydroxyphenyllactic acid | 5,616,804.44 | 9,811,306.00 | 20,001,359.10 | 30,068,826.33 |
| Decanoylcarnitine | 981,156.90 | 739,076.64 | 685,214.66 | 1,095,911.79 |
| N-Acetyl-L-aspartic acid | 929,884.93 | 1,135,564.29 | 1,957,605.69 | 2,501,394.72 |

Panel 2: Sugar alcohols and derivatives-tryptophan metabolism-catecholamines derivatives such as pentose phosphates, N-Acetyl-L-alanine, pentose alcohols, N-Acetyl-Asp-Glu, L-saccharopine, D-Galacturonic acid, quinolinic acid, beta-Pseudouridine, 4-Acetamidobutanoic acid, D-glucuronic acid, 4-Hydroxy-3-methoxyphenylglycol sulfate, D-Threitol, disaccharides, mevalonic acid, orotidine. These metabolites indicate changes in intracellular glucose utilization -, and quinolinic acid - a precursor for NAD⁺ biosynthesis.

**Table 3. Panel 2: Sugar alcohols-tryptophan metabolism-acetylated amino acids associated with ACLF.**

| **Metabolites** | **Healthy subjects (HS)** | **Compensated Cirrhosis (CC)** | **Acute Descompensation (AD)** | **Acute-on-chronic liver failure (ACLF)** |
|---|---|---|---|---|
| Pentose phosphates | 3,255.07 | 6,168.89 | 31,545.67 | 154,120.05 |
| N-Acetyl-L-alanine | 2,136,747.89 | 2,554,828.93 | 3,604,097.36 | 5,468,082.32 |
| Pentose alcohols | 626,222.32 | 1,032,211.73 | 1,710,311.31 | 3,177,392.01 |
| N-Acetyl-Asp-Glu | 9,379.63 | 7,404.77 | 10,561.03 | 26,020.56 |
| L-saccharopine | 261.00 | 193.36 | 3,522.77 | 17,986.95 |
| D-Galacturonic acid | 1,059,862.08 | 2,208,450.24 | 7,247,420.98 | 19,292,643.16 |
| Quinolinic acid | 381,473.29 | 485,343.27 | 1,123,100.26 | 3,859,769.83 |
| beta-Pseudouridine | 2,425,386.80 | 3,590,629.61 | 5,138,511.63 | 8,306,298.88 |
| 4-Acetamidobutanoic acid | 157,809.58 | 204,115.19 | 399,114.17 | 1,216,964.37 |
| D-glucuronic acid | 971,559.69 | 1,026,281.60 | 2,057,058.50 | 4,318,120.06 |
| 4-Hydroxy-3-methoxyphenylglycol sulfate | 34,827.51 | 101,787.93 | 165,336.93 | 375,442.52 |
| D-Threitol | 762,877.76 | 1,176,894.79 | 1,904,765.58 | 4,813,496.08 |
| Disaccharides | 180,140.37 | 232,315.22 | 485,504.39 | 1,371,114.61 |
| Mevalonic acid | 211,928.99 | 240,275.10 | 326,477.96 | 788,266.12 |
| Orotidine | 8,289.89 | 42,263.32 | 73,502.05 | 228,476.20 |

### EXAMPLE 3: Characterization of patient's metabolic profile associated with organ failure.

The aim of the study was also to characterize the metabolic profile associated with each of the three main organ failures (liver, brain, and kidney) and to identify potential pathophysiological mechanisms of the syndrome.

The results shows that the intensity of the ACLF-associated fingerprint increased across ACLF grades, and was similar in patients with kidney failure and in those without, indicating that it was not a simple reflect of decreased kidney excretion but also of cell metabolism alterations.

**Table 4. Panel 1: Acylcarnitines-sialic acid-acetylated amino acids associated with organ failure.**

| **Metabolites** | **Liver** | **Brain** | **Kidney** |
|---|---|---|---|
| 2-Heptanone | 98,992.23 | 191,920.75 | 182,368.58 |
| 5'-Deoxy-5'-(methylthio)adenosine | 108,346.73 | 49,165.71) | 62,135.25 |
| Cystathionine | 108,603.40 | 95,271.31 | 121,518.64 |
| Hexanoylcarnitine | 502,050.10 | 464,633.82 | 521,480.90 |
| N-Acetyl-L-tryptophan | 259,627.55 | 122,917.68 | 7,073,962.07 |
| Phenol | 431,705.25 | 218,629.11 | 295,610.49 |
| N-Acetylneuraminic acid | 313,947.42 | 410,655.78 | 543,069.86 |
| N-Acetyl-L-phenylalanine | 206,743.33 | 180,377.65 | 251,772.50 |
| 2,2'-Thiodiacetic acid | 179,421.63 | 220,414.09 | 226,137.15 |
| Kynurenic acid | 216,935.45 | 268,977.19 | 510,832.58 |
| Octanoylcarnitine | 1,115,690.56 | 943,751.44 | 1,320,218.13 |
| Trisaccharides | 67,081.61 | 143,093.56 | 92,594.65 |
| Hydroxyphenylacetic acids | 1,118,654.81 | 1,390,016.59 | 2,479,024.85 |
| N6,N6,N6-Trimethyl-L-lysine | 379,376.75 | 533,440.85 | 533,440.85 |
| N-Acetyl-L-tyrosine | 460,285.48 | 218,965.02 | 242,132.89 |
| Butyrylcarnitine | 647,576.51 | 460,694.91 | 566,721.79 |
| p-Hydroxyphenyllactic acid | 37,963,319.88 | 31,717,007.84 | 31,592,966.43 |
| Decanoylcarnitine | 949,444.65 | 1,026,450.19 | 1,595,490.82 |
| N-Acetyl-L-aspartic acid | 2,545,608.96 | 2,791,767.31 | 2,755,585.57 |

**Table 5. Panel 2: Sugar alcohols-tryptophan metabolism-acetylated amino acids associated with organ failure.**

| **Metabolites** | **Liver** | **Brain** | **Kidney** |
|---|---|---|---|
| Pentose phosphates | 239,091.96 | 133,333.06 | 154,031.72 |
| N-Acetyl-L-alanine | 5,330,065.47 | 5,330,065.47 | 6,336,103.34 |
| Pentose alcohols | 2,705,199.46 | 2,996,695.88 | 4,198,282.76 |
| N-Acetyl-Asp-Glu | 27,076.14 | 31,652.65 | 30,946.18 |
| L-saccharopine | 21,445.07 | 26,921.02 | 26,711.85 |
| D-Galacturonic acid | 42,216,132.01 | 19,292,643.16 | 18,518,197.47 |
| Quinolinic acid | 3,591,748.14 | 3,503,072.73 | 6,045,655.27 |
| beta-Pseudouridine | 6,826,860.67 | 7,571,529.05 | 10,904,738.69 |
| 4-Acetamidobutanoic acid | 811,810.49 | 854,712.95 | 1,988,168.14 |
| D-glucuronic acid | 10,276,993.30 | 4,344,702.83 | 3,927,654.16 |
| 4-Hydroxy-3-methoxyphenylglycol sulfate | 436,967.02 | 464,457.96 | 477,879.50 |
| D-Threitol | 3,139,408.55 | 3,139,408.55 | 7,718,304.92 |
| Disaccharides | 1,150,975.00 | 1,678,934.43 | 2,263,222.27 |
| Mevalonic acid | 557,830.06 | 761,733.65 | 1,141,329.15 |
| Orotidine | 124,612.64 | 137,710.53 | 444,886.40 |

According to these two panels, ACLF can be distinguishable from AD by a blood metabolite fingerprint capturing blood metabolite accumulation in ACLF and witnessing profound multiorgan alterations in major metabolic pathways, in particular depressed energy production, which may partake into the development of organ failures. In addition, metabolites accumulated in blood may contribute to the severity via toxic effects.

## Claims

1. *Ex vivo* method for diagnostic and/or prognostic of the acute-on-chronic liver failure (ACLF) syndrome in a patient with a liver disorder **characterized in that** it comprises the steps of:
a) measuring a panel of metabolites related with acylcarnitines-sialic acid-acetylated amino acids and/or sugar alcohols and derivatives-tryptophan metabolism-catecholamines derivatives in a biological sample of said patient; and
b) comparing the level of said metabolites in the sample with the level of said metabolites in healthy patients; and
wherein an increase of at least 1.2 times of the level of said metabolites is indicative of ACLF syndrome.

2. Method according to claim 1, **characterized in that** said acylcarnitines-sialic acid-acetylated amino acids are 2-Heptanone, 5'-Deoxy-5'-(methylthio)adenosine, cystathionine, hexanoylcarnitine, N-Acetyl-L-tryptophan, phenol, N-Acetylneuraminic acid, N-Acetyl-L-phenylalanine, 2,2'-Thiodiacetic acid, kynurenic acid, octanoylcarnitine, trisaccharides, hydroxyphenylacetic acids, N6,N6,N6-Trimethyl-L-lysine, N-Acetyl-L-tyrosine, butyrylcarnitine, p-Hydroxyphenyllactic acid, decanoylcarnitine, N-Acetyl-L-aspartic acid.

3. Method according to claim 1, **characterized in that** said sugar alcohols and derivatives-tryptophan metabolism-catecholamines derivatives are pentose phosphates, N-Acetyl-L-alanine, pentose alcohols, N-Acetyl-Asp-Glu, L-saccharopine, D-Galacturonic acid, quinolinic acid, beta-Pseudouridine, 4-Acetamidobutanoic acid, D-glucuronic acid, 4-Hydroxy-3-methoxyphenylglycol sulfate, D-Threitol, disaccharides, mevalonic acid, orotidine.

4. Method according to any one of the preceding claims, **characterized in that** in step a) said biological sample is saliva, whole blood, plasma, serum, or urine of a patient.

5. Method according to claim 4, **characterized in** said biological sample is plasma or serum.

6. Method according to any one of the preceding claims, **characterized in that** said metabolites predict the development of organ failure.

7. Method according to claim 6, **characterized in that** said organ is liver, brain or kidney.

8. Method according to claim 6 or 7, **characterized in that** an increased level of 5'-Deoxy-5'-(methylthio)adenosine, phenol, N-Acetyl-L-tyrosine, butyrylcarnitine, p-Hydroxyphenyllactic acid, pentose phosphates, D-Galacturonic acid or D-glucuronic acid is indicative of liver failure.

9. Method according to claim 6 to 8, **characterized in that** an increased level of 2-Heptanone, trisaccharides, N-Acetyl-L-aspartic acid, N-Acetyl-Asp-Glu or L-saccharopine, is indicative of brain failure.

10. Method according to claim 6 to 9, **characterized in that** an increased level of cystathionine, hexanoylcarnitine, N-Acetyl-L-tryptophan, N-Acetylneuraminic acid, N-Acetyl-L-phenylalanine, 2,2'-Thiodiacetic acid, kynurenic acid, octanoylcarnitine, hydroxyphenylacetic acids, N6,N6,N6-Trimethyl-L-lysine, decanoylcarnitine, N-Acetyl-L-alanine, pentose alcohols, quinolinic acid, beta-Pseudouridine, 4-Acetamidobutanoic acid, 4-Hydroxy-3-methoxyphenylglycol sulfate, D-Threitol, disaccharides, mevalonic acid or orotidine is indicative of kidney failure.
